# EUROPÄISCHE PATENTSCHRIFT

(11) **EP 2 427 134 B1**
(45) Veröffentlichungstag und Bekanntmachung des Hinweises auf die Patenterteilung: **16.01.2013**
(21) Anmeldenummer: 10721411.6
(22) Anmeldetag: 07.05.2010
(51) Int. Cl.: A61C 1/14, B23B 31/02, A61B 17/16

(54) **MEDIZINISCHES HANDSTÜCK**
MEDICAL HAND-PIECE
PIÈCE À MAIN MÉDICALE

(30) Priorität: 07.05.2009 EP 09006196
(43) Veröffentlichungstag der Anmeldung: 14.03.2012
(73) Patentinhaber: W & H Dentalwerk Bürmoos GmbH, 5111 Bürmoos (AT)
(72) Erfinder: WAGNER, Hannes, A-5023 Salzburg (AT); TEUFELBERGER, Gunther, A-5111 Bürmoos (AT); SPITZAUER, Josef, A-5110 Oberndorf (AT)
(74) Vertreter: Benda, Ralf
(86) Internationale Anmeldenummer: PCT/EP2010/056236
(87) Internationale Veröffentlichungsnummer: WO 2010/128131

(56) Entgegenhaltungen:
- EP-A1- 0 820 734
- DE-A1- 3 012 240
- US-A- 5 584 689

## Beschreibung

Die vorliegende Erfindung betrifft ein medizinisches, insbesondere dentales, Handstück mit einer Spannvorrichtung nach dem Oberbegriff des Anspruchs 1. Ein solches Handstück ist in der US-A-5,584,689 offenbart.

Ein weiteres Handstück mit einer derartigen Spannvorrichtung ist zum Beispiel aus der Patentanmeldung US 2009/0220911 A1 bekannt. Die Spannvorrichtung ist dazu ausgebildet, das Werkzeug mit zwei unterschiedlichen Einstecktiefen aufzunehmen, so dass die Länge jenes Teils des Werkzeugs, der aus der Spannvorrichtung (und damit aus dem Handstück) ragt, veränderbar ist. Die Spannvorrichtung weist dazu zwei entlang der Längsachse angeordnete, voneinander beabstandete und unabhängige Abschnitte auf: Einen am oberen Ende der Spannvorrichtung vorgesehenen, länglichen Abschnitt zur Drehmomentübertragung auf das Werkzeug und zur Lagerung und Zentrierung des Werkzeugs in der Spannvorrichtung und einen durch Federlaschen gebildeten, radial federnden Abschnitt zur axialen Sicherung des Werkzeugs in der Spannvorrichtung.

Der Nachteil dieser Spannvorrichtung ist, dass, insbesondere wenn das Werkzeug jene Position einnimmt, in der es weiter aus der Spannvorrichtung herausragt, nur ein sehr kurzer Endabschnitt des Schafts in dem Abschnitt zur Drehmomentübertragung aufgenommen ist, so dass das Werkzeug nur unzureichend in der Spannvorrichtung gelagert und zentriert wird (siehe zum Beispiel Figur 11 der US 2009/0220911 A1). Damit besteht insbesondere bei hohen Drehzahlen die Gefahr, dass das Werkzeug nicht rund läuft oder zu schwingen beginnt.

Der vorliegenden Erfindung liegt daher die Aufgabe zugrunde ein medizinisches, insbesondere dentales, Handstück mit einer Spannvorrichtung zu schaffen, die dazu ausgebildet, das Werkzeug mit zwei unterschiedlichen Einstecktiefen aufzunehmen, wobei das Werkzeug insbesondere in jener Position, in der es weiter aus der Spannvorrichtung herausragt, besser in der Spannvorrichtung gelagert und zentriert werden soll.

Zur Lösung dieser Aufgabe wird ein medizinisches, insbesondere dentales, Handstück vorgeschlagen, das umfasst: Eine Werkzeughalte-/Lösevorrichtung mit einer in eine Antriebsbewegung versetzbare Hohlwelle zur Aufnahme eines Behandlungswerkzeugs, wobei die Hohlwelle sich entlang einer Mittelachse erstreckt und eine Werkzeugaufnahmeöffnung aufweist, eine erste Halteeinheit und eine von der ersten Halteeinheit separate und in Bezug auf die Mittelachse axial von der ersten Halteeinheit beabstandete zweite Halteeinheit, wobei beide Halteeinheiten dazu ausgebildet sind, ein in der Hohlwelle aufgenommenes Behandlungswerkzeug axial zu sichern und ein Drehmoment auf das Behandlungswerkzeug zu übertragen, und wobei beide Halteeinheiten zumindest jeweils ein Formelement und eine die Außenwand der Hohlwelle durchsetzende Bohrung aufweisen, in welcher das zumindest eine Formelement derart aufnehmbar ist, dass es durch die Bohrung in das Innere der Hohlwelle ragt, eine mit den Formelementen zusammenwirkende, relativ zur Hohlwelle bewegbare und die Hohlwelle umgebende Verriegelungshülse und ein mit der Verriegelungshülse zusammenwirkendes Betätigungselement zum Bewegen oder Verschieben der Verriegelungshülse.

Durch diese Ausgestaltung des Handstücks, insbesondere durch die beiden separaten und beabstandeten Halteeinheiten mit ihren Formelementen und Bohrungen in der Hohlwelle, ist es möglich die Hohlwelle über ihre gesamte Länge zur Lagerung und Zentrierung des Behandlungswerkzeugs zu verwenden.

Der Begriff Handstück umfasst alle geraden oder pistolenförmigen Handstücke oder Handgriffe, gebogene Handstücke oder Handgriffe, die im Dentalbereich oftmals als Winkelstücke bezeichnet werden, als auch Teile von Handstücken, insbesondere einen Kopfabschnitt eines Handstücks, der zum Beispiel mit davon lösbaren Griffabschnitten verbindbar ist. Unter der Bezeichnung Handstück werden des Weiteren sowohl schnurlose Handstücke verstanden, insbesondere mit einer austauschbaren oder aufladbaren Energiequelle, als auch Handstücke, die eine Versorgungsleitung und eine damit verbundene Steuer-, Regel- und / oder Versorgungseinheit umfassen.

Um die Lagerung und Zentrierung des Behandlungswerkzeugs noch weiter zu verbessern ist gemäß einem bevorzugten Ausführungsbeispiel die Hohlwelle als hohlzylindrischer, bevorzugt einteiliger, Schaft ausgebildet, dessen Innendurchmesser im Wesentlichen über seine gesamte Länge gleichbleibend ist.

Um die Bedienung der Werkzeughalte-/Lösevorrichtung besonders komfortabel und leichtgängig zu gestalten, weist gemäß einem anderen Ausführungsbeispiel die Verriegelungshülse an ihrer Innenseite einen ersten Rücksprung, welcher der ersten Halteeinheit zugeordnet ist, und einen zweiten Rücksprung, welcher der zweiten Halteeinheit zugeordnet ist, auf, so dass das zumindest eine Formelemente der ersten Halteeinheit in dem ersten Rücksprung und das zumindest eine Formelemente der zweiten Halteeinheit in dem zweiten Rücksprung aufnehmbar ist.

Gemäß einem bevorzugten Ausführungsbeispiel weist der erste Rücksprung eine näher zur Werkzeugaufnahmeöffnung angeordnete Schulter und eine entfernter zur Werkzeugaufnahmeöffnung angeordnete Schulter und der zweite Rücksprung eine näher zur Werkzeugaufnahmeöffnung angeordnete Schulter und eine entfernter zur Werkzeugaufnahmeöffnung angeordnete Schulter auf, wobei der Abstand zwischen den beiden näher zur Werkzeugaufnahmeöffnung angeordneten Schultern der beiden Rücksprünge größer ist als der Abstand zwischen dem Mittelpunkt des zumindest einen Formelements der ersten Halteeinheit und dem Mittelpunkt des zumindest einen Formelements der zweiten Halteeinheit. Dadurch wird sichergestellt, dass, unabhängig ob ein Behandlungswerkzeug in der Hohlwelle aufgenommen ist oder nicht oder wie tief das Behandlungswerkzeug in die Hohlwelle eingesteckt ist, die Formelemente immer ausreichend in oder an der ihnen zugeordneten Bohrung der Hohlwelle fixiert sind.

Gemäß einem anderen bevorzugten Ausführungsbeispiel ist der erste Rücksprung weiter entfernt von der Werkzeugaufnahmeöffnung angeordnet als der zweite Rücksprung, wobei der (die Hohlwelle durchsetzende) Durchmesser des ersten Rücksprungs geringer ist als der Durchmesser des zweiten Rücksprungs. Dadurch wird erreicht, dass das zumindest eine Formelement des ersten Rücksprungs weiter in die Hohlwelle ragt als das zumindest eine Formelement des zweiten Rücksprungs, wodurch das zumindest eine Formelement des ersten Rücksprungs zusätzlich als axialer Anschlag für das Behandlungswerkzeug dient, wenn das Behandlungswerkzeug vollständig in die Hohlwelle eingesteckt ist.

Gemäß einem weiteren bevorzugten Ausführungsbeispiel ist die bezogen auf die Mittelachse axiale Höhe der beiden Rücksprünge unterschiedlich, wobei bevorzugt die axiale Höhe des ersten, weiter entfernt von der Werkzeugaufnahmeöffnung angeordneten Rücksprungs geringer ist als die axiale Höhe des zweiten, näher zur Werkzeugaufnahmeöffnung angeordneten Rücksprungs.

Gemäß einem anderen Ausführungsbeispiel sind die Hohlwelle und die Verriegelungshülse von einer Lagerhülse umgeben, die an ihrem dem Betätigungselement zugewandten Ende eine Öffnung aufweist, durch welche ein Kontakt zwischen der Verriegelungshülse und dem Betätigungselement herstellbar ist. Bevorzugt ragt ein Fortsatz der Verriegelungshülse durch die Öffnung der Lagerhülse. Besonders bevorzugt weist der Fortsatz der Verriegelungshülse ein dem Betätigungselement zugewandtes kugeliges Ende auf, wodurch ein im Wesentlichen punktförmiger Kontakt mit dem Betätigungselement herstellbar ist. Aufgrund des im Wesentlichen punktförmigen Kontakts wird die Übertragung von Wärme auf das Betätigungselement und die damit verbundene Verbrennungsgefahr, wenn das Betätigungselement mit Gewebe in Berührung kommt, deutlich reduziert.

Gemäß einem Ausführungsbeispiel ist an der Lagerhülse ein Antriebselement vorgesehen, um die Hohlwelle in eine Antriebsbewegung zu versetzen. Das Antriebselement ist zum Beispiel als Zahnrad, als Ritzel, als durch ein Druckgas betreibbares Laufrad oder als Teil eines Exzentergetriebes ausgebildet.

Gemäß einem Ausführungsbeispiel ist die Verriegelungshülse durch ein Federelement vorgespannt, das an einer an der Außenseite der Hohlwelle vorgesehenen Schulter oder an einer an der Innenseite der Lagerhülse vorgesehenen Schulter gelagert ist, wodurch eine besonders Platz sparende Ausgestaltung der Werkzeughalte-/Lösevorrichtung erzielt wird.

Gemäß einem weiteren Ausführungsbeispiel ist das zumindest eine Formelement zumindest einer Halteeinheit kugelig ausgebildet oder weist ein kugeliges Ende auf, welches der Mittelachse der Hohlwelle zugewandt ist. Das zumindest eine Formelement ist zum Beispiel als Kugel, Halbkugel, Ellipsoid oder als Zylinder mit kugeligem Ende ausgebildet.

Um eine gleichmäßige und sichere Verspannung des Werkzeugs in der Hohlwelle zu erreichen, sind gemäß einem Ausführungsbeispiel pro Halteeinheit mehrere Formelemente vorgesehen, die, insbesondere gleichmäßig, um die Hohlwelle angeordnet. Bevorzugt weisen die erste Halteeinheit und die zweite Halteeinheit jeweils drei oder sechs Formelemente auf. Die jeweils drei Formelemente werden bevorzugt bei einem durch ein Druckgas betreibbaren Handstück, bei dem geringere Drehmomente übertragen werden, eingesetzt. Bei mechanisch betriebenen Handstücken, die oftmals ausgebildet sind, höhere Drehmomente zu übertragen und bei denen es dem gemäß von Vorteil ist, das Behandlungswerkzeug fester in der Hohlwelle zu fixieren, werden bevorzugt pro Halteeinheit mehr als jeweils drei Formelemente verwendet, besonders bevorzugt jeweils sechs Formelemente.

Gemäß einem Ausführungsbeispiel weist das Handstück ein Behandlungswerkzeug mit einem Behandlungsabschnitt und einem Schaftabschnitt auf, an dem ein einziges Kontaktelement zum wahlweisen Kontakt mit dem zumindest einem Formelement der ersten Halteeinheit oder der zweiten Halteeinheit vorgesehen ist, wobei das Kontaktelement unmittelbar an jenem Ende des Schaftabschnitts angeordnet ist, das am weitesten von dem Behandlungsabschnitt entfernt ist. Damit ist in vorteilhafter Weise ein Behandlungswerkzeug geschaffen, das sowohl mit der erfindungsgemäßen Werkzeughalte-/Lösevorrichtung als auch mit anderen, bekannten Spannvorrichtungen verwendbar ist, insbesondere mit bekannten kraftschlüssigen Spannvorrichtungen mit radial federnden, am Behandlungswerkzeug angreifenden Spannlaschen. Das Behandlungswerkzeug ist lösbar mit dem Handstück verbindbar.

Besonders bevorzugt weist der sich von dem Kontaktelement in Richtung des Behandlungsabschnitts erstreckende, in die Hohlwelle aufnehmbare Teil des Schaftabschnitts einen im Wesentlichen gleichbleibenden Außendurchmesser auf, wodurch die Lagerung und Zentrierung des Behandlungswerkzeugs noch weiter verbessert ist.

Die Erfindung wird nachfolgend anhand bevorzugter Ausführungsbeispiele und Bezug nehmend auf die beigefügten Zeichnungen erläutert:
Figur 1 zeigt ein erstes Ausführungsbeispiel eines medizinischen, mechanisch betreibbaren Handstücks mit einer Werkzeughalte-/Lösevorrichtung, welche eine erste und eine zweite, separate Halteeinheit für das Werkzeug aufweist.
Figur 2 zeigt ein zweites Ausführungsbeispiel eines medizinischen, durch ein Fluid betreibbaren Handstücks mit einer Werkzeughalte-/Lösevorrichtung, welche eine erste und eine zweite, separate Halteeinheit für das Werkzeug aufweist.

Die Figur 3A - 3C zeigen die Werkzeughalte-/Lösevorrichtung der Figur 1 sowie ein darin aufgenommenes Behandlungswerkzeug in unterschiedlichen Einstecktiefen.

Die Figur 3D zeigt die Werkzeughalte-/Lösevorrichtung der Figur 1 ohne Behandlungswerkzeug.

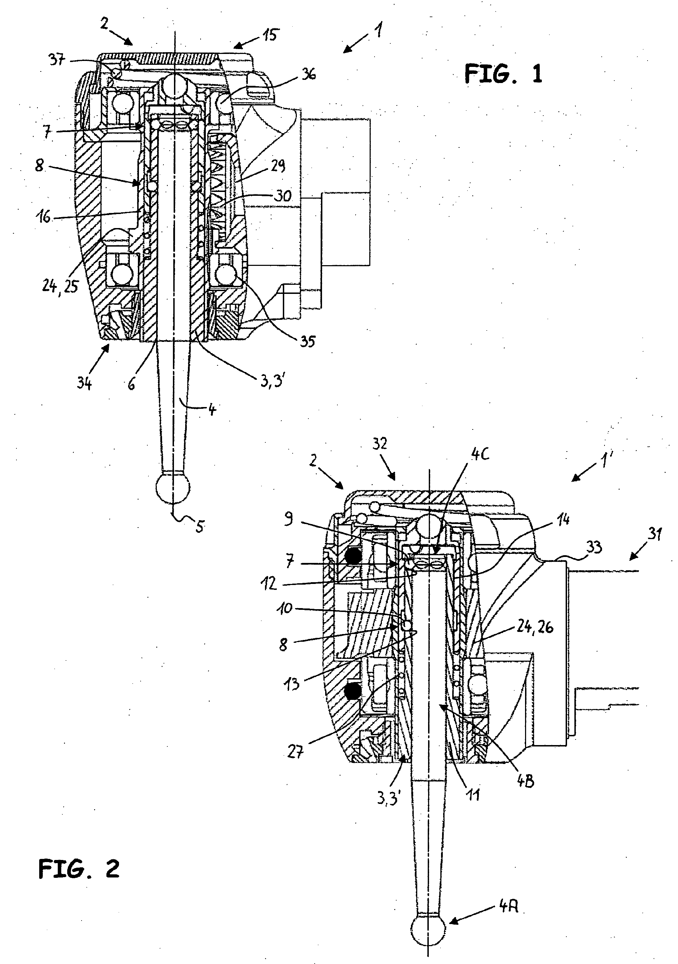

Die Figuren 1 und 2 zeigen zwei medizinische, insbesondere dentale, Handstücke 1, 1 die sich durch die Art des Antriebs unterscheiden: Das Handstück 1 ist als mechanisch betreibbares Handstück ausgebildet, dessen Hohlwelle 3 zur Aufnahme eines Behandlungswerkzeugs 4 über einen mechanischen Antriebszug in eine Antriebsbewegung, insbesondere in eine Drehbewegung, versetzbar ist. Der mechanische Antriebszug umfasst zumindest eine Welle 29, die an einem Ende ein Zahnrad 30 aufweist, das mit einem als Zahnrad oder Ritzel 25 ausgebildeten Antriebselement 24 in Eingriff steht, welches mit der Hohlwelle 3 verbunden ist. Die Welle 29 ist direkt oder indirekt mit einem motorischen Antrieb verbunden oder verbindbar, welcher eine Antriebsbewegung erzeugt, die über die Welle 29, das Zahnrad 30 und das Antriebselement 24 auf die Hohlwelle 3 übertragbar ist.

Das Handstück 1' ist als durch ein Fluid, insbesondere durch ein Druckgas, betreibbares Handstück ausgebildet, bei dem die Hohlwelle 3 zur Aufnahme eines Behandlungswerkzeugs 4 mit einem als Laufrad 26 ausgebildeten Antriebselement 24 verbunden ist. Das Laufrad 26 wird mit einem Fluid beaufschlagt und dadurch gemeinsam mit der Hohlwelle 3 in Bewegung versetzt.

Selbstverständlich kann das Antriebselement 24 auch anders ausgebildet sein, zum Beispiel als Teil eines Exzentergetriebes oder als Schwingungen übertragender Schaft oder als Antriebselement zur Übertragung Sägebewegungen.

Die folgende Beschreibung bezieht sich auf beide Handstücke 1, 1' und auf Bauteile, die bei beiden Handstücken 1, 1' im Wesentlichen gleich sind:

Die Handstücke 1, 1' sind als so genannte Winkelstücke ausgebildet, deren Außenhülse 33 einen Griffabschnitt 31 und einen Kopfabschnitt 32 aufweist. Die Außenhülse 33 kann ein- oder mehrteilig aufgebaut sein. Im oder am Kopfabschnitt 32 sind unter anderem eine Werkzeughalte-/Lösevorrichtung 2, das Antriebselement 24 sowie eine Vorrichtung 34 zur Abgabe eine Flüssigkeit oder eines Flüssigkeits-Druckgas-Gemisches vorgesehen.

Die Werkzeughalte-/Lösevorrichtung 2 umfasst die Hohlwelle 3, die sich entlang einer Mittelachse 5 durch den Kopfabschnitt 32 erstreckt. Die Mittelachse 5 verläuft quer zum Griffabschnitt 31, insbesondere in etwa rechtwinkelig zum Griffabschnitt 31. Falls die Hohlwelle 3 drehbar in dem Handstück 1, 1' gelagert ist, ist bevorzugt die Mittelachse 5 gleich der Rotationsachse der Hohlwelle 3. In die Hohlwelle 3 ist ein Behandlungswerkzeug 4 lösbar einbringbar, zum Beispiel ein Bohrer, eine Säge oder eine Feile. Die Hohlwelle 3 ist als hohlzylindrischer, einteiliger Schaft 3' ausgebildet ist, dessen Innendurchmesser im Wesentlichen über seine gesamte Länge gleichbleibend ist. An einem Ende weist die Hohlwelle 3 eine Werkzeugaufnahmeöffnung 6 auf, die an einer Öffnung der Außenhülse 33 endet oder diese durchsetzt. Durch diese beiden Öffnungen ist ein Behandlungswerkzeug 4 lösbar in die Hohlwelle 3 und damit in den Kopfabschnitt 32 des Handstücks 1, 1' einbringbar bzw. daraus wieder entfernbar.

Mit der Hohlwelle 3 ist drehfest eine Lagerhülse 16 verbunden, zum Beispiel durch Verpressen und / oder durch Verschweißen und / oder durch Verkleben. An der Lagerhülse 16 sind ein oder mehrere Lager, zum Beispiel Wälzlager, insbesondere zwei Kugellager 35, 36, vorgesehen, so dass die Lagerhülse 16 und alle direkt oder indirekt mit der Lagerhülse 16 verbundenen Bauteile, insbesondere die Hohlwelle 3 und eine im Weiteren noch beschriebene Verriegelungshülse 14, relativ zur Außenhülse 33 bewegbar, insbesondere drehbar, sind.

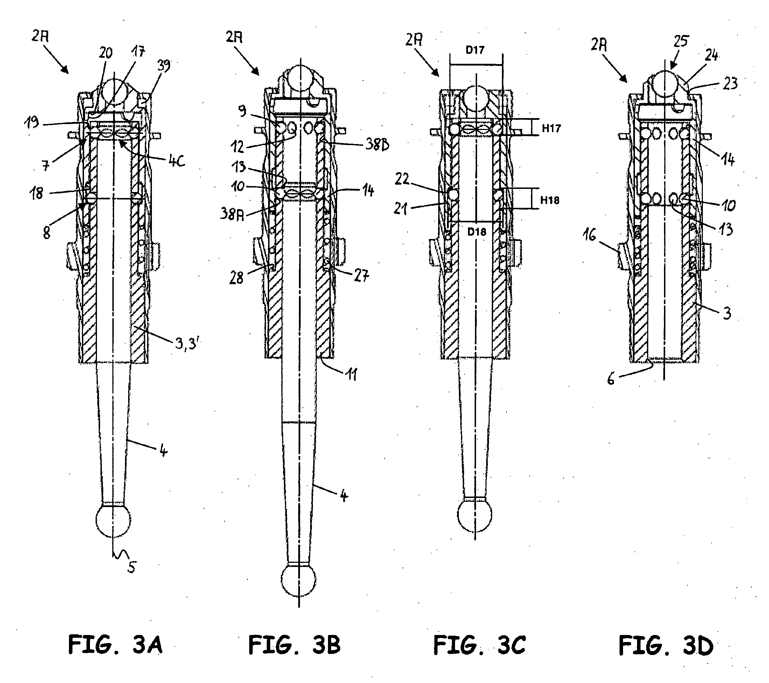

An der Hohlwelle 3 sind als Teil der Werkzeughalte-/Lösevorrichtung 2 eine erste Halteeinheit 7 und eine zweite Halteeinheit 8 vorgesehen, wobei jede der beiden Halteeinheiten 7, 8 dazu ausgebildet ist, ein in der Hohlwelle 3 aufgenommenes Behandlungswerkzeug 4 axial in der Hohlwelle 3 zu sichern und ein vom Antriebselement 24 übertragenes Drehmoment auf das Behandlungswerkzeug 4 weiterzuleiten. Jede der beiden Halteeinheiten 7, 8 ist bevorzugt ausgebildet, die axiale Sicherung des Behandlungswerkzeugs 4 und die Übertragung des Drehmoments unabhängig von der anderen Halteeinheit 7, 8 zu bewerkstelligen. Des Weiteren ist bevorzugt jede der beiden Halteeinheiten 7, 8 ausgebildet, die axiale Sicherung des Behandlungswerkzeugs 4 und die Übertragung des Drehmoments alleine zu bewerkstelligen, d.h. jede Halteeinheit 7, 8 sichert das Behandlungswerkzeug 4 axial und überträgt das Drehmoment auf das Behandlungswerkzeugs 4 ohne wesentliche Mitwirkung der anderen Halteeinheit 7, 8. Dies ist insbesondere aus der Figur 3B gut zu erkennen, wo die axiale Sicherung und die Übertragung des Drehmoments auf das Behandlungswerkzeug 4 ausschließlich durch die zweite Halteeinheit 8 erfolgt. Gleiches gilt jedoch auch für die Figur 3A, in der die erste Halteeinheit 7 das Behandlungswerkzeug 4 axial sichert und das Drehmoment auf das Behandlungswerkzeug 4 überträgt. Unter axialer Sicherung des Behandlungswerkzeugs 4 wird verstanden, dass das Behandlungswerkzeug 4 derart fest in der Hohlwelle 3 aufgenommen oder befestigt ist, dass es von alleine bzw. ungewollt nicht entlang der Mittelachse 5 aus der Hohlwelle 3 gleitet.

Jede der beiden Halteeinheiten 7, 8 weist jeweils zumindest ein, bevorzugt mehrere, Formelemente 9, 10 und eine, bevorzugt mehrere, die Außenwand 11 der Hohlwelle 3 durchsetzende Bohrungen 12, 13 auf, in welcher das zumindest eine Formelement 9, 10 derart aufnehmbar ist, dass es durch die Bohrung 12, 13 in das Innere der Hohlwelle 3 ragt. Die Formelemente 9, 10 sind bevorzugt als Kugeln ausgebildet. Die Bohrungen 12, 13 sind bevorzugt als (bezogen auf die Mittelachse 5) Querbohrungen ausgebildet, die insbesondere den Außenmantel 11 der zylindrisch geformten Hohlwelle 3 durchsetzen. Die Bohrungen 12, 13 sind derart ausgebildet, dass ein Teil der Formelemente 9, 10 in den Innenraum der Hohlwelle 3 ragt, jedoch nicht das gesamte Formelement 9, 10 durch die Bohrungen 12, 13 in den Innenraum der Hohlwelle 3 gelangt. Dies wird zum Beispiel dadurch erreicht, dass die Bohrungen 12, 13 sich in Richtung der Mittelachse 5 etwas verjüngen oder der Durchmesser (parallel zur Mittelachse 5) der Bohrungen 12, 13 sich in Richtung der Mittelachse 5 etwas verringert. Der Durchmesser der Bohrungen 12, 13 ist somit im der Mittelachse zugewandten Bereich der Bohrungen 12, 13 geringer als der Durchmesser der Formelemente 9, 10. Im Gegensatz dazu ist der Durchmesser der Bohrungen 12, 13 an der der Verriegelungshülse 14 zugewandten Öffnung der Bohrungen 12, 13 gleich groß oder größer dem Durchmesser der Formelemente 9, 10.

Bei dem mechanisch betreibbaren Handstück 1 der Figur 1 sind pro Halteeinheit 7, 8 jeweils sechs Formelemente 9, 10 und sechs Bohrungen 12, 13 vorgesehen. Bei dem durch ein Fluid betreibbaren Handstück 1' der Figur 2 sind pro Halteeinheit 7, 8 jeweils drei Formelemente 9, 10 und drei Bohrungen 12, 13 vorgesehen.

Zwischen der Hohlwelle 3 und der Lagerhülse 16, insbesondere in einem Ringspalt zwischen der Hohlwelle 3 und der Lagerhülse 16, ist die Verriegelungshülse 14 angeordnet. Die, bevorzugt ebenfalls hohlzylindrisch ausgebildete, Verriegelungshülse 14 umgibt die Hohlwelle 3 und ist relativ zur Hohlwelle 3 beweglich, insbesondere entlang der Mittelachse 5 verschiebbar. Die Verriegelungshülse 14 ist durch ein Federelement 27, insbesondere eine Spiralfeder, von der Werkzeugaufnahmeöffnung 6 weg oder in Richtung eines Betätigungselements 15 zum Lösen des Behandlungswerkzeugs 4 aus der Hohlwelle 3 vorgespannt. Das Federelement 27 ist an einer an der Außenseite der Hohlwelle 3 vorgesehenen Schulter 28 gelagert (siehe Figur 3B). Anschließend an die Schulter 28 ist die Wandstärke des Mantels der Hohlwelle 3 größer als im Bereich der beiden Halteeinheiten 7, 8. In dem Bereich mit der größeren Wandstärke ist die Hohlwelle 3 direkt mit der Lagerhülse 16 verbunden.

Die Verriegelungshülse 14 weist an ihrer der Mittelachse 5 zugewandten Innenseite einen ersten Rücksprung 17 auf, welcher im Bereich der ersten Halteeinheit 7 angeordnet ist und / oder der ersten Halteeinheit 7 zugeordnet ist. Die Verriegelungshülse 14 weist des Weiteren einen zweiten Rücksprung 18 auf, welcher im Bereich der zweiten Halteeinheit 8 angeordnet ist und / oder der zweiten Halteeinheit 8 zugeordnet ist. Die Rücksprünge 17, 18 sind somit derart angeordnet und derart bemessen, dass das zumindest eine Formelement 9 der ersten Halteeinheit 7 in dem ersten Rücksprung 17 und das zumindest eine Formelement 10 der zweiten Halteeinheit 8 in dem zweiten Rücksprung 18 aufnehmbar ist. Die Tiefe der Rücksprünge 17, 18 ist derart bemessen, dass, wenn ein Formelement 9, 10 zumindest teilweise darin aufgenommen ist, das Formelement 9, 10 nicht oder weniger weit in die Hohlwelle 3 ragt. Ist ein Formelement 9, 10 nicht in einem Rücksprung 17, 18 aufgenommen, sondern kontaktiert einen an einen Rücksprung 17, 18 anschließenden Innenwandabschnitt 38A, 38B der Verriegelungshülse 14, dann ragt das Formelement 9, 10 in die Hohlwelle 3 oder weiter in die Hohlwelle 3. Die Rücksprünge 17, 18 sind bevorzugt als entlang der Innenseite der Verriegelungshülse 14 verlaufende Ringnuten oder Kreisbögen ausgebildet.

Beide Rücksprunge 17, 18 weisen jeweils eine näher zur Werkzeugaufnahmeöffnung 6 angeordnete Schulter 19, 21 und eine entfernter zur Werkzeugaufnahmeöffnung 6 angeordnete Schulter 20, 22 auf (siehe Figuren 3A und 3C). Der Abstand zwischen den beiden näher zur Werkzeugaufnahmeöffnung 6 angeordneten Schultern 19, 21 der beiden Rücksprünge 17, 18 ist größer als der Abstand zwischen dem Mittelpunkt des zumindest einen Formelements 9 der ersten Halteeinheit 7 und dem Mittelpunkt des zumindest einen Formelements 10 der zweiten Halteeinheit 8. Die Differenz zwischen dem Abstand der beiden Schultern 19, 21 und dem Abstand der Mittelpunkte der beiden Formelemente 9, 10 beträgt zum Beispiel etwa 0,3 - 1,0 mm, bevorzugt etwa 0,4 - 0,5 mm. Der Abstand zwischen den beiden Schultern 19, 21 beträgt zum Beispiel etwa 2,0 - 4,0 mm, der Abstand zwischen den Mittelpunkten der beiden Formelemente 9, 10 beträgt zum Beispiel etwa 1,5 - 3,5 mm.

Der (quer zur Mittelachse 5 verlaufende) Durchmesser D17 des ersten Rücksprungs 17, der weiter entfernt von der Werkzeugaufnahmeöffnung 6 angeordnet ist als der zweite Rücksprung 18, ist geringer als der (quer zur Mittelachse 5 verlaufende) Durchmesser D18 des zweiten Rücksprungs 18. Durch den geringeren Durchmesser D17 werden die Formelemente 9 näher zur Mittelachse 5 bewegt oder ragen weiter in den Innenraum der Hohlwelle 3 hinein. Dadurch bilden das oder die Formelemente 9 des ersten Rücksprungs 17 zusätzlich einen axialen Anschlag für das Behandlungswerkzeug 4 bzw. für eine im Endbereich des Schaftabschnitts 4B des Behandlungswerkzeugs 4 vorgesehene Schulter (siehe Figur 3A). Bevorzugt liegt die Differenz zwischen den beiden Durchmesser D17, D18 im Bereich von etwa 0,25 - 0,05 mm, insbesondere bei etwa 0,1 mm. Der Durchmesser D17 des ersten Rücksprungs 17 beträgt zum Beispiel in etwa 2,3 - 2,6 mm, bevorzugt in etwa 2,5 mm. Der Durchmesser D18 des zweiten Rücksprungs 18 beträgt zum Beispiel in etwa 2,4 - 2,7 mm, bevorzugt in etwa 2,6 mm.

Die beiden Rücksprünge 17, 18 weisen des Weiteren bezogen auf die Mittelachse 5 axialen Höhen H17, H18 mit unterschiedlich Abmessungen auf. Bevorzugt ist die axiale Höhe H17 des ersten, weiter entfernt von der Werkzeugaufnahmeöffnung 6 angeordneten Rücksprungs 17 geringer ist als die axiale Höhe H18 des zweiten, näher zur Werkzeugaufnahmeöffnung 6 angeordneten Rücksprungs 18. Die Höhen H17, H18 der beiden Rücksprünge 17, 18 betragen zum Beispiel in etwa 0,5 - 1,5 mm, die Differenz zwischen den beiden Höhen H17, H18 liegt zum Beispiel bei 0,2 - 0,5 mm.

Die Verriegelungshülse 14 weist an ihrem dem Betätigungselement 15 zugewandten Ende einen Fortsatz 24 auf, der durch eine Öffnung 23 der Lagerhülse 16 ragt (siehe insbesondere Figur 3D). Der Fortsatz 24 hat ein kugeliges Ende 25, das entweder als integraler Teil der Verriegelungshülse 14 ausgebildet ist oder durch ein separates Bauteil, zum Beispiel eine Kugel oder Halbkugel, gebildet ist, das mit dem Fortsatz 24 verbunden ist, zum Beispiel in einem Sitz verpresst oder drehbar aufgenommen ist. Das kugelige Ende 25 dient zum, insbesondere im Wesentlichen nur punktförmigen, Kontakt mit dem Betätigungselement 15, über das der Anwender die Verriegelungshülse 14 verschieben kann und somit ein Behandlungswerkzeug 4 in der Hohlwelle 3 befestigen und / oder daraus lösen kann.

Das zum Beispiel als Druckkappe oder Druckdeckel ausgebildete Betätigungselement 15 ist durch ein Federelement 37, insbesondere durch eine Spiralfeder, vorgespannt (siehe Figur 1) und verschiebbar, insbesondere relativ zur Hohlwelle 3, zur Verriegelungshülse 14 und zur Lagerhülse 16, am Kopfabschnitt 32 des Handstücks 1, 1' angebracht. Wird das Betätigungselement 15 entgegen der Federkraft des Federelements 37 in Richtung der Verriegelungshülse 14 verschoben, so kontaktiert es das kugelige Ende 25 der Verriegelungshülse 14 und verschiebt im Weiteren die Verriegelungshülse 14 entgegen der Federkraft des Federelements 27. Wird das Betätigungselement 15 los gelassen, so verschieben die beiden Federelemente 27, 37 die Verriegelungshülse 14 bzw. das Betätigungselement 15 selbsttätig wieder in die in der Figur 1 dargestellte Ausgangsposition. Bevorzugt weist das Betätigungselement 15 an seiner Innenseite einen Fortsatz auf, der dem kugeligen Ende 25 gegenüber liegt und über das der Kontakt mit dem kugeligen Ende 25 herstellbar ist.

Ein bevorzugter Aufbau eines Behandlungswerkzeugs 4, das mit dem Handstück 1, 1' verbindbar ist und in die Werkzeughalte-/Lösevorrichtung 2 einbringbar ist, ist zum Beispiel in der Figur 2 dargestellt: Das Behandlungswerkzeug 4 umfasst einen Behandlungsabschnitt 4A und einen Schaftabschnitt 4B. Der Behandlungsabschnitt 4A ist ausgebildet, eine Behandlungsstelle zum Abtrag von Material oder Gewebe zu kontaktieren und ist mit Klingen oder Schneiden versehen. Der Schaftabschnitt 4B dient zur Verbindung mit dem Handstück 1, 1'. Zumindest ein Teil des Schaftabschnitts 4B ist bevorzugt zylindrisch ausgebildet. An dem Schaftabschnitt 4B ist bevorzugt ein einziges Kontaktelement 4C zum wahlweisen Kontakt mit dem zumindest einem Formelement 9, 10 der ersten Halteeinheit 7 oder der zweiten Halteeinheit 8 vorgesehen. Besonders bevorzugt ist das Kontaktelement 4C unmittelbar an jenem Ende des Schaftabschnitts 4B angeordnet, das am weitesten von dem Behandlungsabschnitt 4A entfernt ist. Das Kontaktelement 4C umfasst zum Beispiel zumindest eine Aufnahme, einen Einstich, einen Rücksprung, eine Ringnut oder eine Vertiefungen, zum Eingriff des zumindest einen Formelements 9, 10. Das Kontaktelement 4C kann zum Beispiel den Schaftabschnitt 4B ringförmig oder halbkreisförmig umgeben und / oder Kalotten aufweisen. Der an das einzige Kontaktelement 4C anschließende, sich in Richtung des Behandlungsabschnitts 4A erstreckende und in die Hohlwelle 3 aufnehmbare Teil des Schaftabschnitts 4B weist einen im Wesentlichen gleichbleibenden Außendurchmesser auf.

Die Figuren 3A - 3D zeigen die Spanvorrichtung 2A, die Teil der Werkzeughalte-/Lösevorrichtung 2 ist. Die Figur 3A zeigt das Behandlungswerkzeug 4 in seiner zurückgezogenen Position, in welcher es weniger weit aus dem Handstück 1, 1' bzw. aus der Werkzeughalte-/Lösevorrichtung 2 ragt. Die axiale Sicherung und die Drehmomentübertragung des Behandlungswerkzeugs 4 erfolgt durch die erste Halteeinheit 7, dessen Formelemente 9 aus dem ersten Rücksprung 17 ausgetreten sind und durch einen an den Rücksprung 17 anschließenden Innenwandabschnitt 38B (siehe Figur 3B) der Verriegelungshülse 14 in den Innenraum der Hohlwelle 3 gedrängt werden, wo sie in das Kontaktelement 4C des Behandlungswerkzeugs 4 eingreifen. Die Formelemente 10 der zweiten Halteeinheit 8 sind zumindest teilweise im zweiten Rücksprung 18 aufgenommen und kontaktieren den Schaftabschnitt 4B des Behandlungswerkzeugs 4 nicht oder nur geringfügig, insbesondere nur an einem Kontaktpunkt. Des Weiteren ist zu erkennen, dass die näher zur Werkzeugaufnahmeöffnung 6 angeordnete Schulter 21 des zweiten Rücksprungs 18 die Formelemente 10 kontaktiert, so dass die Formelemente 10 als Anschlag für die Schulter 21 dienen und die Bewegung der Verriegelungshülse 14 in Richtung des Betätigungselements 15 begrenzen. Die Höhe H18 des zweiten Rücksprungs 18 ist bevorzugt derart bemessen, dass die Schulter 21 an den Formelementen 10 anschlägt, ohne dass die Verriegelungshülse 14 mit ihrer dem Betätigungselement 15 zugewandten Seite die Lagerhülse 16 kontaktiert, so dass zwischen der Verriegelungshülse 14 und der Lagerhülse 16 ein kleiner Freiraum 39 entsteht bzw. der Fortsatz 24 der Verriegelungshülse 14 nicht mit seiner maximal möglichen Länge aus der Öffnung 23 ragt.

Die Figur 3B zeigt das Behandlungswerkzeug 4 in seiner vorgeschobenen Position, in welcher es weit aus dem Handstück 1, 1' bzw. aus der Werkzeughalte-/Lösevorrichtung 2 ragt. Die axiale Sicherung und die Drehmomentübertragung des Behandlungswerkzeugs 4 erfolgt durch die zweite Halteeinheit 8, dessen Formelemente 10 aus dem zweiten Rücksprung 18 ausgetreten sind und durch einen an den Rücksprung 18 anschließenden Innenwandabschnitt 38A der Verriegelungshülse 14 in den Innenraum der Hohlwelle 3 gedrängt werden, wo sie in das Kontaktelement 4C des Behandlungswerkzeugs 4 eingreifen. Die Formelemente 9 der ersten Halteeinheit 7 werden durch einen an den Rücksprung 18 anschließenden Innenwandabschnitt 38B der Verriegelungshülse 14 ebenfalls in den Innenraum der Hohlwelle 3 gedrängt, der jedoch in diesem Bereich leer ist, d.h. in dem sich kein Teil des Schaftabschnitts 4B des Behandlungswerkzeugs 4 befindet. In dieser Positon kontaktiert die Verriegelungshülse 14 mit ihrer dem Betätigungselement 15 zugewandten Seite die Innenseite der Lagerhülse 16 und der Fortsatz 24 der Verriegelungshülse 14 ragt mit seiner maximal möglichen Länge aus der Öffnung 23.

Die Figur 3C zeigt eine Situation, in der das Behandlungswerkzeug 4 in die Werkzeughalte-/Lösevorrichtung 2 eingeschoben oder herausgezogen wird. Mit Hilfe des Betätigungselements 15 ist die Verriegelungshülse 14 entgegen der Federkraft des Federelements 27 in die Lagerhülse 16 oder in Richtung der Werkzeugaufnahmeöffnung 6 geschoben. Die Formelemente 9, 10 beider Halteeinheiten 7, 8 sind zumindest teilweise in ihren jeweiligen Rücksprüngen 17, 18 der Verriegelungshülse 14 aufgenommen, kontaktieren den Schaftabschnitt 4B des Behandlungswerkzeugs 4 nicht oder nur geringfügig und greifen insbesondere nicht in das Kontaktelement 4C des Behandlungswerkzeugs 4 ein, so dass der Schaftabschnitt 4B des Behandlungswerkzeugs 4 ohne großen Widerstand in der Hohlwelle 3 verschiebbar ist.

Insbesondere aus den Figuren 3A und 3B ist somit zu erkennen, dass die Handstücke 1, 1' oder die Werkzeughalte-/Lösevorrichtung 2, d.h. insbesondere die Halteeinheiten 7, 8 und die Verriegelungshülse 14, derart ausgebildet sind, dass das Behandlungswerkzeug 4 mit unterschiedlichen Einstecktiefen in der Hohlwelle 3 befestigbar ist, wobei die Einstecktiefen (d.h. die Position des Behandlungswerkzeugs 4 in der Hohlwelle 3 oder die Länge des aus dem Handstück 1, 1` ragenden Teils des Behandlungswerkzeugs 4) durch die Halteeinheiten 7, 8 vorbestimmt oder definiert sind. Es ist des Weiteren zu erkennen, dass die Handstücke 1, 1' oder die Werkzeughalte-/Lösevorrichtung 2, d.h. insbesondere die Halteeinheiten 7, 8 und die Verriegelungshülse 14, derart ausgebildet sind, dass die axiale Sicherung des Behandlungswerkzeugs 4 und die Übertragung des Drehmoments jeweils nur durch einer der beiden Halteeinheiten 7, 8 erfolgt, d.h. dass jeweils nur eine der beiden Halteeinheit 7, 8 ohne wesentliche Mitwirkung der anderen Halteeinheit 7, 8 das Behandlungswerkzeug 4 axial sichert und das Drehmoment auf das Behandlungswerkzeug 4 überträgt.

Es sind alle Merkmale aller beschriebenen und dargestellten Ausführungsbeispiele miteinander kombinierbar. So ist es insbesondere möglich, die Werkzeughalte-/Lösevorrichtung 2 mit mehr als zwei Halteeinheiten 7, 8 zu versehen, so dass das Behandlungswerkzeug 4 in mehr als zwei unterschiedlichen Einstecktiefen in der Hohlwelle 3 fixierbar ist. Des Weiteren können im Sinne einer kinetischen Umkehrung die Verriegelungshülse 14 und das Federelement 27 derart angeordnet sein, dass die Verriegelungshülse 14 in Richtung der Werkzeugaufnahmeöffnung 6 oder von dem Betätigungselement 15 weg vorgespannt ist.

## Patentansprüche

1. Medizinisches, insbesondere dentales, Handstück (1, 1') umfassend eine Werkzeughalte-/Lösevorrichtung (2) mit einer in eine Antriebsbewegung versetzbaren Hohlwelle (3) zur Aufnahme eines Behandlungswerkzeugs (4), wobei die Hohlwelle (3) sich entlang einer Mittelachse (5) erstreckt und eine Werkzeugaufnahmeöffnung (6) aufweist, aufweisend
- eine erste Halteeinheit (7) und eine von der ersten Halteeinheit (7) separate und in Bezug auf die Mittelachse (5) axial von der ersten Halteeinheit (7) beabstandete zweite Halteeinheit (8), wobei beide Halteeinheiten (7, 8) dazu ausgebildet sind, ein in der Hohlwelle (3) aufgenommenes Behandlungswerkzeug (4) axial zu sichern und ein Drehmoment auf das Behandlungswerkzeug (4) zu übertragen, und wobei beide Halteeinheiten (7, 8) jeweils zumindest ein Formelement (9, 10) und eine die Außenwand (11) der Hohlwelle (3) durchsetzende Bohrung (12, 13) aufweisen, in welcher das zumindest eine Formelement (9, 10) derart aufnehmbar ist, dass es durch die Bohrung (12, 13) in das Innere der Hohlwelle (3) ragt,
- eine mit den Formelementen (9, 10) zusammenwirkende, relativ zur Hohlwelle (3) bewegbare und die Hohlwelle (3) umgebende Verriegelungshülse (14) und
- ein mit der Verriegelungshülse (14) zusammenwirkendes Betätigungselement (15) zum Bewegen oder Verschieben der Verriegelungshülse (14).

2. Medizinisches, insbesondere dentales, Handstück (1, 1') nach Anspruch 1, **dadurch gekennzeichnet, dass**
die Hohlwelle (3) als hohlzylindrischer, bevorzugt einteiliger, Schaft (3') ausgebildet ist, dessen Innendurchmesser im Wesentlichen über seine gesamte Länge gleichbleibend ist.

3. Medizinisches, insbesondere dentales, Handstück (1, 1') nach einem der vorherstehenden Ansprüche, **dadurch gekennzeichnet, dass**
die Verriegelungshülse (14) an ihrer Innenseite einen ersten Rücksprung (17), welcher der ersten Halteeinheit (7) zugeordnet ist, und einen zweiten Rücksprung (18), welcher der zweiten Halteeinheit (8) zugeordnet ist, aufweist, so dass das zumindest eine Formelement (9) der ersten Halteeinheit (7) in dem ersten Rücksprung (17) und das zumindest eine Formelement (10) der zweiten Halteeinheit (8) in dem zweiten Rücksprung (18) aufnehmbar ist.

4. Medizinisches, insbesondere dentales, Handstück (1, 1') nach Anspruch 3, **dadurch gekennzeichnet, dass**
der erste Rücksprung (17) eine näher zur Werkzeugaufnahmeöffnung (6) angeordnete Schulter (19) und eine entfernter zur Werkzeugaufnahmeöffnung (6) angeordnete Schulter (20) aufweist, dass der zweite Rücksprung (18) eine näher zur Werkzeugaufnahmeöffnung (6) angeordnete Schulter (21) und eine entfernter zur Werkzeugaufnahmeöffnung (6) angeordnete Schulter (22) aufweist und dass der Abstand zwischen den beiden näher zur Werkzeugaufnahmeöffnung (6) angeordneten Schultern (19, 21) der beiden Rücksprünge (17, 18) größer ist als der Abstand zwischen dem Mittelpunkt des zumindest einen Formelements (9) der ersten Halteeinheit (7) und dem Mittelpunkt des zumindest einen Formelements (10) der zweiten Halteeinheit (8).

5. Medizinisches, insbesondere dentales, Handstück (1, 1') nach Anspruch 3 oder 4, **dadurch gekennzeichnet, dass**
der erste Rücksprung (17) weiter entfernt von der Werkzeugaufnahmeöffnung (6) angeordnet ist als der zweite Rücksprung (18), wobei der Durchmesser (D17) des ersten Rücksprungs (17) geringer ist als der Durchmesser (D18) des zweiten Rücksprungs (18).

6. Medizinisches, insbesondere dentales, Handstück (1, 1') nach einem der Ansprüche 3 - 5, **dadurch gekennzeichnet, dass**
die bezogen auf die Mittelachse (5) axiale Höhe (H17, H18) der beiden Rücksprünge (17, 18) unterschiedlich ist, wobei bevorzugt die axiale Höhe (H17) des ersten, weiter entfernt von der Werkzeugaufnahmeöffnung (6) angeordneten Rücksprungs (17) geringer ist als die axiale Höhe (H18) des zweiten, näher zur Werkzeugaufnahmeöffnung (6) angeordneten Rücksprungs (18).

7. Medizinisches, insbesondere dentales, Handstück (1, 1') nach einem der vorherstehenden Ansprüche, **dadurch gekennzeichnet, dass**
die Hohlwelle (3) und die Verriegelungshülse (14) von einer Lagerhülse (16) umgeben sind, die an ihrem dem Betätigungselement (15) zugewandten Ende eine Öffnung (23) aufweist, durch welche ein Kontakt zwischen der Verriegelungshülse (14) und dem Betätigungselement (15) herstellbar ist.

8. Medizinisches, insbesondere dentales, Handstück (1, 1') nach Anspruch 7, **dadurch gekennzeichnet, dass**
ein Fortsatz (24) der Verriegelungshülse (14) durch die Öffnung (23) der Lagerhülse 16) ragt.

9. Medizinisches, insbesondere dentales, Handstück (1, 1') nach Anspruch 8, **dadurch gekennzeichnet, dass**
der Fortsatz (24) der Verriegelungshülse (14) ein dem Betätigungselement (15) zugewandtes kugeliges Ende (25) aufweist, wodurch ein im Wesentlichen punktförmiger Kontakt mit dem Betätigungselement (15) herstellbar ist.

10. Medizinisches, insbesondere dentales, Handstück (1, 1') nach einem der Ansprüche 7 - 9, **dadurch gekennzeichnet, dass**
an der Lagerhülse (16) ein Antriebselement (24, 25, 26) vorgesehen ist, um die Hohlwelle (3) in eine Antriebsbewegung zu versetzen.

11. Medizinisches, insbesondere dentales, Handstück (1, 1') nach einem der vorherstehenden Ansprüche, **dadurch gekennzeichnet, dass**
die Verriegelungshülse (14) durch ein Federelement (27) vorgespannt ist, das an einer an der Außenseite der Hohlwelle (3) vorgesehenen Schulter (28) oder an einer an der Innenseite der Lagerhülse (16) vorgesehenen Schulter gelagert ist.

12. Medizinisches, insbesondere dentales, Handstück (1, 1') nach einem der vorherstehenden Ansprüche, **dadurch gekennzeichnet, dass**
das zumindest eine Formelement (9, 10) zumindest einer Halteeinheit (7, 8) kugelig ausgebildet ist oder ein kugeliges Ende aufweist, welches der Mittelachse (5) der Hohlwelle (3) zugewandt ist.

13. Medizinisches, insbesondere dentales, Handstück (1, 1') nach einem der vorherstehenden Ansprüche, **dadurch gekennzeichnet, dass**
die erste Halteeinheit (7) und die zweite Halteeinheit (8) jeweils drei oder sechs Formelemente (9, 10) aufweisen.

14. Medizinisches, insbesondere dentales, Handstück (1, 1') nach einem der vorherstehenden Ansprüche, **gekennzeichnet durch**
ein Behandlungswerkzeug (4), das einen Behandlungsabschnitt (4A) und einen Schaftabschnitt (4B) aufweist, an dem ein einziges Kontaktelement (4C) zum wahlweisen Kontakt mit dem zumindest einem Formelement (9, 10) der ersten Halteeinheit (7) oder der zweiten Halteeinheit (8) vorgesehen ist, wobei das Kontaktelement (4C) unmittelbar an jenem Ende des Schaftabschnitts (4B) angeordnet ist, das am weitesten von dem Behandlungsabschnitt (4A) entfernt ist.

15. Medizinisches, insbesondere dentales, Handstück (1, 1') nach Anspruch 14, **dadurch gekennzeichnet, dass**
der sich von dem einzigen Kontaktelement (4C) in Richtung des Behandlungsabschnitts (4A) erstreckende, in die Hohlwelle (3) aufnehmbare Teil des Schaftabschnitts (4B) einen im Wesentlichen gleichbleibenden Außendurchmesser aufweist.

## Claims

1. Medical, particularly dental, handpiece (1, 1') comprising a tool holding/releasing device (2) with a hollow shaft (3) for holding a treatment tool (4) that can be placed in a drive movement, wherein the hollow shaft (3) extends along a central axis (5) and has a tool receptacle opening (6), comprising
- a first holding unit (7) and a second holding unit (8) separate from the first holding unit (7) and axially offset from the first holding unit (7) with respect to the central axis (5), wherein both holding units (7, 8) are configured to axially secure a treatment tool (4) supported in the hollow shaft (3) and to transmit a torque to the treatment tool (4), and wherein each holding unit (7, 8) comprises at least one shaped element (9, 10) and a bore (12, 13) penetrating the outer wall (11) of the hollow shaft (3), in which the at least one shaped element (9, 10) can be held in such a way that it projects through the bore (12, 13) into the interior of the hollow shaft (3),
- a locking sleeve (14) operatively interacting with the shaped elements (9, 10) that can move relative to the hollow shaft (3) and that surrounds the hollow shaft (3) and
- an operating element (15) for the movement or sliding of the locking sleeve (14) that operatively interacts with the locking sleeve (14).

2. The medical, in particular dental, handpiece (1, 1') according to claim 1, **characterized in that**
the hollow shaft (3) is configured as a hollow cylindrical, preferably single-part, shaft (3') whose inner diameter is substantially constant along its entire length.

3. The medical, in particular dental, handpiece (1, 1') according to any one of the preceding claims, **characterized in that**
the locking sleeve (14) has a first recess (17) on its inner side which is associated with the first holding unit (7) and a second recess (18) which is associated with the second holding unit (8), so that the at least one shaped element (9) of the first holding unit (7) can be held in the first recess (17) and the at least one shaped element (10) of the second holding unit (8) can be held in the second recess (18).

4. The medical, in particular dental, handpiece (1, 1') according to claim 3, **characterized in that**
the first recess (17) has a shoulder (19) located closer to the tool receptacle opening (6) and a shoulder (20) located more distant from the tool receptacle opening (6), that the second recess (18) has a shoulder (21) located closer to the tool receptacle opening (6) and a shoulder (22) located more distant from the tool receptacle opening (6), and that the distance between the two shoulders (19, 21) of the two recesses (17, 18) closer to the tool receptacle opening (6) is greater than the distance between the centre point of at least one shaped element (9) of the first holding unit (7) and the centre point of at least one shaped element (10) of the second holding unit (8).

5. The medical, in particular dental, handpiece (1, 1') according to claim 3 or 4, **characterized in that**
the first recess (17) is located further from the tool receptacle opening (6) than the second recess (18), wherein the diameter (D17) of the first recess (17) is less than the diameter (D18) of the second recess (18).

6. The medical, in particular dental, handpiece (1, 1') according to any one of claims 3 - 5, **characterized in that**
the axial height (H17, H18) of the two recesses (17, 18) relative to the central axis (5) is different, wherein preferably the axial height (H17) of the first recess (17) located further from the tool receptacle opening (6) is less than the axial height (H18) of the second recess (18) located closer to the tool receptacle opening (6).

7. The medical, in particular dental, handpiece (1, 1') according to any one of the preceding claims, **characterized in that**
the hollow shaft (3) and the locking sleeve (14) are surrounded by a bearing sleeve (16) that has an opening (23) on its end facing the operating element (15) through which contact can be made between the locking sleeve (14) and the operating element (15).

8. The medical, in particular dental, handpiece (1, 1') according to claim 7, **characterized in that**
a protrusion (24) of the locking sleeve (14) projects through the opening (23) of the bearing sleeve (16).

9. The medical, in particular dental, handpiece (1, 1') according to claim 8, **characterized in that**
the protrusion (24) of the locking sleeve (14) has a spherical end (25) facing the operating element (15), by means of which a substantially point-shaped contact can be achieved with the operating element (15).

10. The medical, in particular dental, handpiece (1, 1') according to any one of claims 7 - 9, **characterized in that**
a drive element (24, 25, 26) is provided on the bearing sleeve (16) in order to place the hollow shaft (3) in a drive motion.

11. The medical, in particular dental, handpiece (1, 1') according to any one of the preceding claims, **characterized in that**
the locking sleeve (14) is tensioned by a spring element (27) that is supported on a shoulder (28) located on the outer side of the hollow shaft (3) or a shoulder located on the inner side of the bearing sleeve (16).

12. The medical, in particular dental, handpiece (1, 1') according to any one of the preceding claims, **characterized in that**
the at least one shaped element (9, 10) of at least one holding unit (7, 8) is spherical or has a spherical end that faces the central axis (5) of the hollow shaft (3).

13. The medical, in particular dental, handpiece (1, 1') according to any one of the preceding claims, **characterized in that**
the first holding unit (7) and the second holding unit (8) each have three or six shaped elements (9, 10).

14. The medical, in particular dental, handpiece (1, 1') according to any one of the preceding claims, **characterized by**
a treatment tool (4) that has a treatment section (4A) and a shaft section (4B), on which a single contact element (4C) is provided for selective contact with the at least one shaped element (9, 10) of the first holding unit (7) or the second holding unit (8), wherein the contact element (4C) is located directly at that end of the shaft section (4B) that is furthest away from the treatment section (4A).

15. The medical, in particular dental, handpiece (1, 1') according to claim 14, **characterized in that**
the part of the shaft section (4B) which extends from the only contact element (4C) in the direction of the treatment section (4A) and which can be held in the hollow shaft (3) has a substantially constant outer diameter.

## Revendications

1. Pièce à main (1, 1') médicale, en particulier dentaire, comprenant un dispositif de fixation/libération d'outil (2) avec un arbre creux (3) pouvant être mis dans un mouvement d'entraînement pour recevoir un outil de traitement (4), sachant que l'arbre creux (3) s'étend le long d'un axe médian (5) et présente une ouverture de réception d'outil (6), comprenant
- une première unité de fixation (7) et une seconde unité de fixation (8) séparée de la première unité de fixation (7) et distante axialement de la première unité de fixation (7) par rapport à l'axe médian (5), sachant que les deux unités de fixation (7, 8) sont formées pour bloquer axialement un outil de traitement (4) reçu dans l'arbre creux (3) et transmettre un couple de rotation à l'outil de traitement (4) et sachant que les deux unités de fixation (7, 8) présentent respectivement au moins un élément de moulage (9, 10) et un alésage (12, 13) traversant la paroi extérieure (11) de l'arbre creux (3) dans lequel l'au moins un élément de moulage (9, 10) peut être reçu de manière à dépasser dans l'intérieur de l'arbre creux (3) à travers l'alésage (12, 13),
- un manchon de blocage (14) coopérant avec les éléments de moulage (9, 10), mobile par rapport à l'arbre creux (3) et entourant l'arbre creux (3) et
- un élément d'actionnement (15) coopérant avec le manchon de blocage (14) pour déplacer ou pousser le manchon de blocage (14).

2. Pièce à main (1, 1') médicale, en particulier dentaire, selon la revendication 1, **caractérisée en ce que**
l'arbre creux (3) est conçu en tant que manche (3') cylindrique creux, de préférence d'une seule pièce, dont le diamètre intérieur est essentiellement égal sur toute sa longueur.

3. Pièce à main (1, 1') médicale, en particulier dentaire, selon l'une des revendications précédentes, **caractérisée en ce que**
le manchon de blocage (14) présente sur son côté intérieur un premier retour (17), attribué à la première unité de fixation (7), et un deuxième retour (18), attribué à la deuxième unité de fixation (8), de façon à ce que l'au moins un élément de moulage (9) de la première unité de fixation (7) puisse être reçu dans le premier retour (17) et l'au moins un élément de moulage (10) de la seconde unité de fixation (8) puisse être reçu dans le second retour (18).

4. Pièce à main (1, 1') médicale, en particulier dentaire, selon la revendication 3, **caractérisée en ce que**
le premier retour (17) présente un épaulement (19) disposé plus près de l'ouverture de réception d'outil (6) et un épaulement (20) disposé plus loin de l'ouverture de réception d'outil (6), que le second retour (18) présente un épaulement (21) disposé plus près de l'ouverture de réception d'outil (6) et un épaulement (22) disposé plus loin de l'ouverture de réception d'outil (6), et que l'écart entre les deux épaulements (19, 21) disposés plus près de l'ouverture de réception d'outil (6) des deux retours (17, 18), est supérieur à l'écart entre le centre de l'au moins un élément de moulage (9) de la première unité de fixation (7) et le centre de l'au moins un élément de moulage (10) de la seconde unité de fixation (8).

5. Pièce à main (1, 1') médicale, en particulier dentaire, selon la revendication 3 ou 4, **caractérisée en ce que**
le premier retour (17) est disposé encore plus loin de l'ouverture de réception d'outil (6) que le second retour (18), sachant que le diamètre (D17) du premier retour (17) est inférieur au diamètre (D18) du second retour (18).

6. Pièce à main (1, 1') médicale, en particulier dentaire, selon l'une des revendications 3 à 5, **caractérisée en ce que**
la hauteur axiale (H17, H18) des deux retours (17, 18) rapportée à l'axe médian (5) est différente, sachant que de préférence la hauteur axiale (H17) du premier retour (17) disposé encore plus loin de l'ouverture de réception d'outil (6) est inférieure à la hauteur axiale (H18) du second retour (18) disposé plus près de l'ouverture de réception d'outil (6).

7. Pièce à main (1, 1') médicale, en particulier dentaire, selon l'une des revendications précédentes, **caractérisée en ce que**
l'arbre creux (3) et le manchon de blocage (14) sont entourés par un manchon de palier (16) qui présente sur son extrémité tournée vers l'élément d'actionnement (15), une ouverture (23) à travers laquelle un contact peut être créé entre le manchon de blocage (14) et l'élément d'actionnement (15).

8. Pièce à main (1, 1') médicale, en particulier dentaire, selon la revendication 7, **caractérisée en ce qu'**
un appendice (24) du manchon de blocage (14) fait saillie à travers l'ouverture (23) du manchon de palier (16).

9. Pièce à main (1, 1') médicale, en particulier dentaire, selon la revendication 8, **caractérisée en ce que**
l'appendice (24) du manchon de blocage (14) présente une extrémité (25) sphérique tournée vers l'élément d'actionnement (15), ce par quoi un contact essentiellement en forme de point peut être créé avec l'élément d'actionnement (15).

10. Pièce à main (1, 1') médicale, en particulier dentaire, selon l'une des revendications 7 à 9, **caractérisée en ce qu'**
un élément d'entraînement (24, 25, 26) est prévu sur le manchon de palier (16) pour décaler l'arbre creux (3) dans un mouvement d'entraînement.

11. Pièce à main (1, 1') médicale, en particulier dentaire, selon l'une des revendications précédentes, **caractérisée en ce que**
le manchon de blocage (14) est précontraint par un élément de ressort (27) qui est disposé sur un épaulement (28) prévu sur le côté extérieur de l'arbre creux (3) ou un épaulement prévu sur le côté intérieur du manchon de palier (16).

12. Pièce à main (1, 1') médicale, en particulier dentaire, selon l'une des revendications précédentes, **caractérisée en ce que**
l'au moins un élément de moulage (9, 10) d'au moins une unité de fixation (7, 8) est sphérique ou présente une extrémité sphérique, laquelle est tournée vers l'axe médian (5) de l'arbre creux (3).

13. Pièce à main (1, 1') médicale, en particulier dentaire, selon l'une des revendications précédentes, **caractérisée en ce que**
la première unité de fixation (7) et la seconde unité de fixation (8) présentent respectivement trois ou six éléments de moulage (9, 10).

14. Pièce à main (1, 1') médicale, en particulier dentaire, selon l'une des revendications précédentes, **caractérisée par**
un outil de traitement (4) qui présente une section de traitement (4A) et une section de manche (4B), sur lequel un unique élément de contact (4C) est prévu pour le contact au choix avec l'au moins un élément de moulage (9, 10) de la première unité de fixation (7) ou de la seconde unité de fixation (8), sachant que l'élément de contact (4C) est disposé directement sur l'extrémité de la section de manche (4B) qui est la plus éloignée de la section de traitement (4A).

15. Pièce à main (1, 1') médicale, en particulier dentaire, selon la revendication 14, **caractérisée en ce que**
la partie de la section de manche (4B) qui pouvant être reçue dans l'arbre creux (3) et qui s'étendant de l'unique élément de contact (4C) en direction de la section de traitement (4A), présente un diamètre extérieur essentiellement constant.
